# EUROPEAN PATENT APPLICATION

(11) **EP 4 487 868 A1**
(43) Date of publication of application: **08.01.2025**
(21) Application number: 23183973.9
(22) Date of filing: 06.07.2023
(51) Int. Cl.: A61K 39/00, C12N 15/113

(54) **MODIFIED NK CELL EXPRESSING A CHIMERIC ANTIGEN RECEPTOR**

(71) Applicant: Universitätsklinikum Hamburg-Eppendorf, 20246 Hamburg (DE)
(72) Inventor: AYUK, Francis Ayuketang, 20246 Hamburg (DE); FEHSE, Boris, 20246 Hamburg (DE); HARFMANN, Maraike, 22307 Hamburg (DE); GLOW, Dawid, 20246 Hamburg (DE); SCHROEDER, Tanja, 20246 Hamburg (DE)
(74) Representative: Uexküll & Stolberg

(57) **Abstract**

The present invention generally relates to the field of cell therapy. In particular, the invention relates to a modified natural killer (NK) cell which is characterized by the inactivation of the endogenous CD45 and CD38 genes, the expression of a chimeric antigen receptor (CAR) which is directed against CD45 and/or CD38, and the inability to proliferate. The modified NK cell of the invention exerts cytotoxicity against malignant cells upon its contacting with a malignant cell. Accordingly, the modified NK cell of the invention can be applied in cell therapy approaches, in particular for the treatment of cancer or immunological diseases. The cell is also particularly useful for stem cell transplantation. In yet another aspect, the invention relates to a method of producing the above NK cell. The method comprises inactivating the endogenous CD45 and CD38 genes of the NK cell, modifying the NK cell to express a CAR directed against CD45 and/or CD38, and irradiating the NK cell such that it loses its ability to proliferate.

## Description

The present invention generally relates to the field of cell therapy. In particular, the invention relates to a modified natural killer (NK) cell which is characterized by the inactivation of the endogenous CD45 and CD38 genes, the expression of a chimeric antigen receptor (CAR) which is directed against CD45 and/or CD38, and the inability to proliferate. The modified NK cell of the invention exerts cytotoxicity against malignant cells upon its contacting with a malignant cell. Accordingly, the modified NK cell of the invention can be applied in cell therapy approaches, in particular for the treatment of cancer or immunological diseases. The cell is also particularly useful for stem cell transplantation. In yet another aspect, the invention relates to a method of producing the above NK cell. The method comprises inactivating the endogenous CD45 and CD38 genes of the NK cell, modifying the NK cell to express a CAR directed against CD45 and/or CD38, and irradiating the NK cell such that it loses its ability to proliferate.

### BACKGROUND OF THE INVENTION

Chimeric antigen receptor (CAR)-based cell therapy represents a new and very effective therapeutic modality, particularly for malignant hematologic diseases [1,2]. Autologous T cells are most commonly used as effector cells, but there are now also successful approaches using allogeneic NK cells, e.g. derived from umbilical cord blood [3]. Previous clinically successful approaches have used CARs against antigens expressed on the target cells, but not on the effector cells [1-3]. When CARs were used against antigens that are also expressed on T cells, fratricide (i.e., mutual destruction of CAR T cells) was observed and this could be circumvented e.g., by knockout of the respective antigens in the effector cells [4-6]. Such knockout of target antigens on effector cells is only possible if the survival and function of the effector cells are not affected.

In CAR-based therapies, the identification of disease-specific target antigens is challenging. Many of the CARs currently investigated in preclinical and clinical studies bind so-called tumor-associated antigens (TAAs), which are expressed not only on malignant but also on healthy cells, which raises the fear of a considerable ("on-target off-tumor") toxicity of the CAR-T cells. For example, in the case of leukemia, CD45, CD33, CD123 as well as CD38 are being investigated as possible targets for CAR-based therapy. On the other hand, it is known that CD45 is expressed on almost all hematopoietic cells, while CD33 is expressed in the liver [18], CD123 on endothelial cells [19], and CD38 on respiratory epithelial cells and muscle tissue [20]. Therefore, when CAR-T cells are used against such antigens, "safety switches" have been suggested to minimize side effects or to provide better and faster treatment. However, these switches are very elaborate and not always reliable.

CD45 has attracted particular interest as a possible target, as this protein is not expressed on non-hematopoietic cells in the human body. Since CD45 is, however, expressed on T lymphocytes and NK cells, a knockout is required for CAR-therapy. Given the central role of CD45 in the regulation of effector cell activation, signal transduction, and cytotoxicity [7], a significant loss of function of the resulting cells must be expected upon knockout of CD45. This was confirmed in early work with the RNK-16 cell line (rat), in which wild-type cells, but not CD45-negative mutants, were cytotoxically active [8]. In contrast, it was later shown in the mouse model that CD45-negative primary NK cells exhibit defects in activation, signal transduction and cytokine production, whereas cytotoxic activity was preserved in vitro, albeit to a reduced extent [9, 10]. While the mouse data indicate residual functionality of CD45-negative primary NK cells, activation and signal transduction in murine and human NK cells are in part different. For example, murine and human NKG2D differ significantly in structure and the signaling pathways used [11]. Binding and "crosslinking" with anti-CD45 antibodies was found to inhibit the cytotoxic effect of primary human NK cells, but not primary human T cells, although the exact mechanism is yet unclear. The antibodies could stimulate a cytotoxic-responsive function of CD45, or trigger an inhibitory effect [12-15]. Data on functionality of CD45-negative T cells are lacking, mainly because CD45^{-/-} mice develop only very few or no T cells [16]. Indeed, the role and relevance of CD45 does not only differ considerably between species, but also between T and NK cells [17].

The above considerations likewise apply to CD38, a glycoprotein that is present on many of the different types of immune cells in humans. CD38 appears to play an important role in NK cell activation, signal transduction and cytotoxicity [22, 23], which renders it highly questionable whether T cells or NK cells would still be functional after a knockout of both CD45 and CD38.

A further practical problem with therapies using immortalized or malignant cell lines is the need to irradiate the cells that are intended to be used for treatment prior to their administration to a patient. Such irradiation is a prerequisite for clinical application of the cells, as it must be ensured for safety reasons that the ability of the cells to proliferate and multiply in the human body is abolished. However, irradiation significantly reduces or even completely abolishes cytotoxicity of the cells which renders the cells unsuitable for their therapeutic purpose, at least in the treatment of malignant diseases. In view of the foregoing, it was an objective of the invention to develop a novel type of modified immune effector cell which is cytotoxic to malignant cells and can be administered to patients without any safety concerns.

The present invention addresses this need and provides additional advantages as well. In particular, the invention provides a modified cytotoxic NK cell which is CD45-negative and CD38-negative, lacks the ability to proliferate, and expresses a CD45CAR, a CD38CAR or both. The modified NK cell is highly suitable for therapeutic use, in particular for treating malignant diseases, such as leukemia.

### DESCRIPTION OF THE INVENTION

Thus, in a first aspect, the present invention provides a modified natural killer (NK) cell, wherein said cell is characterized by
(a) the inactivation of the endogenous CD45 and CD38 genes,
(b) the expression of a chimeric antigen receptor (CAR) which is directed against CD45 and/or a CAR which is directed against CD38,
(c) the inability to proliferate,
wherein said cell exerts cytotoxicity against malignant cells upon its contacting with a malignant cell. The NK cell preferably is a mammalian cell, and more preferably a human cell.

NK cells are the predominant subgroup of lymphocyte in the mammalian immune system that is responsible for mediating anti-tumor and anti-viral responses. NK cells can be identified by the specific pattern of cell surface markers. Most significantly, NK cell express the surface marker CD56, but are negative for CD3 (CD56⁺ CD3⁻). Any type of NK cell that is known in the art can be used for preparing the modified cell of the invention, including primary NK cells and cell lines. For example, NK cells may be obtained from peripheral blood mononuclear cells (PBMCs) that are isolated from a buffy coat of a whole-blood sample using an appropriate NK isolation kit e.g. Dynabeads^{™} Untouched^{™} Human NK cells (Thermo Fisher Scientific, Schwerte, Germany). If necessary, the activation of the NK cells can be performed by commonly known methods, e.g. by the addition of human IL-2. The cells can also be derived from suitable progenitor cells, such as cord blood, hematopoietic stem cells, or induced pluripotent stem cells [28].

Alternatively, a NK cell line can be used. Several NK cell lines are available from cell collections, such as the Leibniz-Institut DSMZ - Deutsche Sammlung von Mikroorganismen und Zellkulturen (Braunschweig, Germany). A particularly suitable cell line is the KHYG-1 cell line that has been deposited at the Leibniz-Institut DSMZ - Deutsche Sammlung von Mikroorganismen und Zellkulturen under accession number ACC 725. In a preferred aspect, the NK cell to be used for practicing the invention is the cell line KHYG-1 or a cell line derived from KHYG-1. The KHYG-1 cell line has the particular advantage that the cells can be irradiated at a dose of 5-50 Gy to interfere with their ability to proliferate without abolishing their cytotoxicity. Rather, the cells maintain their cytotoxicity for several days after irradiation [21].

Preferably, the NK cell to be used for preparing the modified NK cell of the invention has a point mutation in exon 7 of the p53 gene. The point mutation may be a C to T conversion at nucleotide 877 in codon 248 of the p53 gene, as it can be observed in KHYG-1.

According to the invention, the NK cell has an inactive endogenous CD45 gene and an inactive endogenous CD38 gene. Gene inactivation can be achieved in a number of suitable ways, all of which are known in the art. The gene of interest may be disrupted by the inclusion of an unrelated nucleotide sequence, which causes a shift in the reading frame. Alternatively, part of the nucleotide sequence of the gene may be deleted. Gene inactivation can be achieved, e.g. by homologous recombination or gene editing using suitable designer enzymes, such as CRISPR/CAS, TALENs or others. In a preferred embodiment, the gene inactivation of the CD45 and CD38 genes has been performed by CRISPR/CAS gene editing.

In addition, the modified NK cell of the invention expresses a CAR which is directed against CD45 and/or a CAR which is directed against CD38. In one embodiment, the modified NK cell of the invention expresses a CAR which is directed against CD45, but no CAR which is directed against CD38. In one embodiment, the modified NK cell of the invention expresses a CAR which is directed against CD38, but no CAR which is directed against CD45. In yet another embodiment, the modified NK cell of the invention expresses a CAR which is directed against CD45 and a CAR which is directed against CD38.

At least eight different isoforms of CD45 have been reported in humans. At least six of these isoforms are expressed by different blood cells at reasonable levels [7]. In one embodiment, the CD45CAR targets the RABC isoform of human CD45 (SEQ ID NO:1). In another embodiment, the CD45CAR targets the R0 isoform of human CD45 (SEQ ID NO:2). In yet another embodiment, the CD45CAR targets the RAB isoform of human CD45 (SEQ ID NO:3). In yet another embodiment, the CD45CAR targets the RAC isoform of human CD45 (SEQ ID NO:4). In yet another embodiment, the CD45CAR targets the RBC isoform of human CD45 (SEQ ID NO:5). In yet another embodiment, the CD45CAR targets the RA isoform of human CD45 (SEQ ID NO:6). In yet another embodiment, the CD45CAR targets the RB isoform of human CD45 (SEQ ID NO:7). In yet another embodiment, the CD45CAR targets the RC isoform of human CD45 (SEQ ID NO:8). For CD38, two isoforms have been reported in humans. In one embodiment, the CD38CAR targets the P28907-1 isoform of human CD38 (SEQ ID NO:9). In another embodiment, the CD45CAR targets the P28907-2 isoform of human CD38 (SEQ ID NO:10).

In yet another embodiment, the modified NK cell of the invention expresses a CAR which is directed against CD45 and/or a CAR which is directed against CD38, and an additional CAR which is directed to another protein. The additional CAR is preferably directed to a protein selected from the group consisting of B Cell Maturation Antigen (BCMA), Kappa or lambda light chain, Lewis Y, CLL-1, G protein-coupled receptor, class C group 5 member D (GPRC5D), Fc receptor-homolog 5 (FcRH5), Human Semaphorin-4A (Sema4A) HLA-A1, HLA-A2,TCRalpha/beta, TCR gamma/delta, CD3, CD4, CD5, CD7, CD10, CD11a, CD13, CD16, CD19, CD20, CD22, CD23, CD26, CD30, CD33, CD34, CD37, CD38, CD43, CD44v6, CD45, CD56, CD59, CD79a, CD79b, CD86, CD90, CD115, CD117, CD123, CD133, CD138 and CD319. In a preferred embodiment, the additional CAR is directed to the cell surface protein CD123. In yet another preferred embodiment, the additional CAR is directed to the cell surface protein CD19. In yet another preferred embodiment, the additional CAR is directed to the cell surface protein Sema4A.

The NK cell can be modified to express a CAR in several ways. For example, the genome of the NK cell can be modified by the inclusion of a nucleotide sequence that codes for a CD45CAR and/or a CD38CAR construct using genome-editing techniques. Alternatively, a nucleotide sequence encoding a CD45CAR and/or a CD38CAR construct can be introduced into the NK cell by transfection with a non-viral vector (e.g. a transposon) or by transduction with a viral vector, preferably a retroviral vector (e.g. a γ-retroviral, α-retroviral, or lentiviral vector). A suitable construct for use in the method of the invention is disclosed, for example, in WO2017/222593A1 and comprises an anti-CD45 single-chain variable fragment (scFv), CD8-derived hinge (H) and transmembrane (TM) regions, and tandem CD28 and 4-1BB co-activation domains linked to the CD3 signaling domain. The construct uses a strong spleen focus forming virus promoter (SFFV) and a CD8 leader sequence. Transduction is achieved in WO2017/222593A1 by a lentiviral vector system, as described in the below examples.

The modified NK cell of the invention is suitable for use in medicine. More specifically, the NK cell of the invention is particularly suitable for use in a method of treating a cancer disease or an immunological disease in a patient. When used in cell therapeutic treatment approaches, the NK cell may be autologous or allogeneic in respect of the patient to be treated. In one embodiment, the modified NK cell is autologous in respect of the subj ect that receives the modified cell, i.e. the cell that is modified to provide the modified cell according of the invention has previously been obtained from said subject. In another embodiment, the modified NK cell is allogeneic in respect of the subject that receives the modified cell, i.e. the cell that is modified to provide the modified cell according of the invention has previously been obtained from a genetically non-identical subject that belongs to the same species as the subject that receives the modified cell. It is particularly preferred that the modified NK cell of the invention is an allogeneic cell.

The cancer disease to be treated by the modified NK cells of the invention is preferably selected from the group consisting of leukemia, lymphoma, myeloma, myelodysplastic syndromes, and myeloproliferative neoplasms (MPN).

The immunological disease to be treated by the modified NK cells of the invention is preferably selected from the group consisting of systemic lupus erythematosus, systemic sclerosis, multiple sclerosis, diabetes mellitus type I, aplastic anemia, Addison disease, acquired hemophilia, primary agammaglobulinemia, ankylosing spondylitis, antiphospholipid syndrome, autoimmune encephalitis, autoimmune gastritis, autoimmune hepatitis, autoimmune hemolytic anemia, autoimmune myocarditis, autoimmune oophoritis, autoimmune pancreatitis, autoimmune dermatitis, autoimmune polyglandular syndrome, Balo disease, Behcet's disease, bullous pemphigoid, Crohn's disease, cold agglutinin disease, Cogan's syndrome, celiac disease, CREST syndrome, dermatomyositis, dermatitis herpetiformis, discoid lupus, Grave's disease, Hashimoto thyroiditis, myasthenia gravis, pernicious anemia, Castleman disease, eosinophilic fasciitis, Evan's syndrome, glomerulonephritis, Goodpasture's syndrome, Granulomatosis polyangiitis, Guillain-Barré syndrome, Henoch-Schönlein IgA vasculitis, IgA nephropathy, Immune thrombocytopenia, juvenile idiopathic arthritis, juvenile polymyositis, Lambert-Eaton syndrome, lupus nephritis, Ménière's disease, mixed connective tissue disease, myasthenia gravis, POEMS syndrome, polyarthritis nodosa, polymyalgia rheumatic, polymyositis, pemphigus vulgaris, primary biliary cirrhosis, psoriasis, pure red cell aplasia, rheumatoid arthritis, reactive arthritis, rheumatic fever, sarcoidosis, Sjögren syndrome, ulcerative colitis, undifferentiated connective tissue disease, and vitiligo.

In another embodiment, die modified NK cell of the invention is used for conditioning a subject prior to autologous or allogeneic hematopoietic stem cell transplantation or other cellular therapy, e.g. CAR-T cell therapy. The modified NK cell of the invention may be administered as a single agent or in combination with one or more other agents such as irradiation, cytotoxic drugs or other anti-cancer agents. In this regard, the modified NK cell may be administered simultaneously, before or after the other agent or agents.

In another aspect, the present invention provides a method of producing a modified NK cell which is unable to proliferate and exerts cytotoxicity against malignant cells upon its contacting with the malignant cell, said method comprising
(a) providing an NK cell,
(b) inactivating the endogenous CD45 and CD38 genes of the NK cell,
(c) modifying the NK cell such that it expresses a CAR directed against CD45 and/or a CAR directed against CD38, and
(d) irradiating the NK cell such that it loses its ability to proliferate.

The method of the invention is directed to the manufacturing of a genetically modified NK cell according to the first aspect of the invention which is useful for therapeutic purposes. In a first step of the method, i.e., step (a) of the method of the invention, a NK cells are provided. As outlined above, the cells can be either directly isolated from blood or derived from suitable progenitor cells, such as cord blood, hematopoietic stem cells, or induced pluripotent stem cells or from a cell line. If the cells are intended to be used in a cell therapeutic treatment of a disease, the cell may be autologous in respect of the subject that receives the modified cell, i.e. the cell provided in step (a) of the method of the invention has previously been obtained from said subject. Alternatively, the cell may be allogeneic in respect of the subject that receives the modified cell, i.e. the cell provided in step (a) of the method of the invention has previously been obtained from a genetically non-identical subject that belongs to the same species as the subject that receives the modified cell. It is particularly preferred that the NK cell to be modified by the above manufacturing method of the invention is an autologous cell.

In step (b) of the method, the endogenous CD45 and CD38 genes of the NK cell are inactivated. CD45 and CD38 gene inactivation can be achieved as explained elsewhere herein. Preferably, targeted genome editing, and more preferably CRISPR/CAS or TALENs are used for gene inactivation.

In step (c) of the method, the NK cell is modified to express a CAR directed against CD45 and/or a CAR directed against CD38. Methods for the CAR modification are known in the art and have been discussed above in connection with the description of the modified cells. For example, a nucleotide sequence encoding a CD45CAR and/or a CD38CAR construct can be introduced into a NK cell by transfection with a non-viral vector or by transduction with a viral vector, preferably a retroviral vector, such as a γ-retroviral, α-retroviral, or lentiviral vector. A suitable construct for a nucleotide sequence encoding a CD45CAR and a method for transducing said construct with a lentiviral vector system are disclosed in international patent application WO2017/222593A1. The method preferably comprises modifying the NK cell such that it expresses a CAR directed against CD45 and a CAR directed against CD38.

According to the method of the invention, if the source of the cells is peripheral blood, cord blood or induced pluripotent stem cells, the inactivation step (b) can be performed either before, simultaneous with or after modification step (c). In one embodiment, inactivation step (b) is performed before modification step (c). In another embodiment, inactivation step (b) is performed after modification step (c). In a particularly preferred embodiment, the CAR modification step (c), and preferably the transduction of the cell, is performed after CD45 and CD38 inactivation in step (b). For example, the CAR modification step (c) can be carried out 12-36 hours after performing inactivation step (b). Preferably, the time period between inactivation step (b) and modification step (c) is at least 12 hours, at least 14 hours, at least 16 hours, at least 18 hours, at least 20 hours, at least 22 hours, at least 24 hours, at least 26 hours, at least 28 hours, at least 30 hours, at least 32 hours, or at least 34 hours.

If the source of the cells is an NK cell line, the inactivation step (b) may be performed either before, after or simultaneous with the modification step (c). Thereby, the inactivation of CD45 and CD38 may be performed separately or simultaneously. Also, the modification step for CD45CAR and CD38CAR may be performed separately or simultaneously. The cells may be cultured, expanded and enriched after each step in order to obtain the desired cell population and number of cells with inactivated CD45 and CD38 and expression of CD45CAR and/or CD38CAR.

The modified NK cell expressing a CAR against CD45 and/or CD38 may be further modified to express one or more additional CARs. For example, the NK cell may express a CAR which is directed to a protein selected from the group consisting of B Cell Maturation Antigen (BCMA), Kappa or lambda light chain, Lewis Y, CLL-1, G protein-coupled receptor, class C group 5 member D (GPRC5D), Fc receptor-homolog 5 (FcRH5), Human Semaphorin-4A (Sema4A), HLA-A1, HLA-A2, TCR alpha/beta, TCR gamma/delta, CD3, CD4, CD5, CD7, CD10, CD11a, CD13, CD16, CD19, CD20, CD22, CD23, CD26, CD30, CD33, CD34, CD37, CD38, CD43, CD44v6, CD45, CD56, CD59, CD79a, CD79b, CD86, CD90, CD115, CD117, CD123, CD133, CD138 and CD319. In a preferred embodiment, the NK cell expresses a CD45-CAR and a CD123-CAR. In another preferred embodiment, the NK cell expresses a CD38-CAR and a CD123-CAR. In yet another preferred embodiment, the NK cell expresses a CD45-CAR, a CD38-CAR and a CD123-CAR. In yet another preferred embodiment, the NK cell expresses a CD45-CAR and a CD19-CAR. In yet another preferred embodiment, the NK cell expresses a CD38-CAR and a CD19-CAR. In yet another preferred embodiment, the NK cell expresses a CD45-CAR, a CD38-CAR and a CD19-CAR. In yet another preferred embodiment, the NK cell expresses a CD38-CAR and a CAR targeting Sema4A.

Alternatively, the modified NK cell may express only a single CAR, e.g. a CD45-CAR, and is used in a mixture of cells in combination with another cell that expresses CD123-CAR. In another embodiment, the NK cell expresses a CD38-CAR and is used in a mixture of cells in combination with another cell that expresses CD123-CAR. In another embodiment, the NK cell expresses a CD45-CAR and is used in a mixture of cells in combination with another cell that expresses a CD38-CAR and another cell that expresses a CD123-CAR. In yet another embodiment, the NK cell expresses a CD38-CAR and is used in a mixture of cells in combination with another cell that expresses a CAR targeting Sema4A. In yet another embodiment, the NK cell expresses a CD45-CAR and is used in a mixture of cells in combination with another cell that expresses a CAR targeting Sema4A. In yet another embodiment, the NK cell expresses a CD45-CAR and is used in a mixture of cells in combination with another cell that expresses a CD19-CAR. In yet another embodiment, the NK cell expresses a CD38-CAR and is used in a mixture of cells in combination with another cell that expresses a CD19-CAR. In yet another embodiment, the NK cell expresses a CD45-CAR and is used in a mixture of cells in combination with another cell that expresses a CD38-CAR and another cell that expresses a CD19-CAR.

The one or more additional CAR may be an activating CAR, which means that it triggers an activating signal of the NK cell (e.g. a proliferation or cytotoxicity signal), or it may be an inhibitory CAR, which means that it triggers an inhibitory signal of the NK cell that leads to reduction or inhibition of proliferation or cytotoxicity, e.g. based on the signaling domain of NK cell inhibitory receptors. This can be achieved, e.g., by transducing the NK cell with separate viral vectors that code for CD45-CAR, CD38-CAR, and for example, HLA-A2-CAR, respectively.

In another embodiment, the NK cells are further modified to reduce recognition und possible rejection by an allogeneic immune system e.g. by knockout or knockdown of HLA molecules which can be achieved e.g. by knockout or knockdown of Beta-2 microgloblulin e.g. using CRISPR/Cas9.

In another embodiment, the NK cells produced by the method of the invention are additionally modified to allow their selective elimination in case of unwanted side effects. These modifications might be based on suitable genetic elements, e.g. pro-drug-converting enzymes or suitable receptor molecules, facilitating cell suicide (apoptosis) after application of corresponding prodrugs or ligands. Alternatively, these modifications might be receptor/cell-surface molecules as potential targets of antibody-dependent cellular cytotoxicity.

In another embodiment, instead of the respective CAR, e.g. the CD45CAR, the immune cells express a universal CAR, whose activity depends on the presence of a soluble adapter, which links the CAR to, e.g., CD45 on the target cell. The generation of a universal CAR (UniCAR) is described, for example, in reference [29].

In step (d) of the method of the invention, the NK cell is irradiated to prevent that the cells proliferate after their administration into a patient, such as a human patient. Irradiation of the NK cells can be carried out either before or after inactivation step (b) and modification step (c). It is however preferred that the irradiation is carried out after steps (a)-(c) have been carried out, and before administering the modified NK cell to the patient. For example, irradiation can be carried out 48 hours, 36 hours, 24 hours, 12 hours, 6 hours, 4 hours, 2 hours, or 1 hour before administration to the patient. Typically, gamma-irradiation with e.g. a cobalt-60 source is used.

Irradiation is performed at a dose to ensure that the cells lose their ability to proliferate and multiply. Typically, the cells are irradiated at a dose of between 5-50 Gy. If the modified cells are cryopreserved, irradiation may be performed before or after cryopreservation. In this regard, irradiation is preferably, performed after cryopreservation and thawing.

After the step (d), the cells can be either administered to a subject in need of the cells or stored until further use, e.g. by freezing. In a particular embodiment, the cells obtained by the method of the invention are cryopreserved, e.g. by freezing the cells. For example, the cells can be frozen to a temperature below -80°C and stored until further use, e.g. by administration into the patient.

The method of the invention may further comprise an additional step (e), which is performed after steps (a)-(c) or (a)-(d) have been carried out, and in which the NK cell is tested for CD45 and CD45-CAR expression and/or for CD38 and CD38-CAR expression. Preferably, testing can occur 2 days, 4 days, 6 days, 8 days, 10 days, 12 days, 14 days, 16 days, 18 days, or 20 days after inactivation step (b) and/or modification step (c). CD45 and CD45-CAR and/or CD38 and CD38-CAR expression can be detected, for example, by using antibodies directed against CD45 and CD45-CAR, respectively. Preferably, CD45 and CD45-CAR expression and/or CD38 and CD38-CAR expression is analyzed by flow cytometry. If the source of the cells is a cell line, step (e) may be performed after steps (a)-(c) or (a)-(d) and before and/or after cryopreservation and thawing.

### BRIEF DESCRIPTION OF THE FIGURES

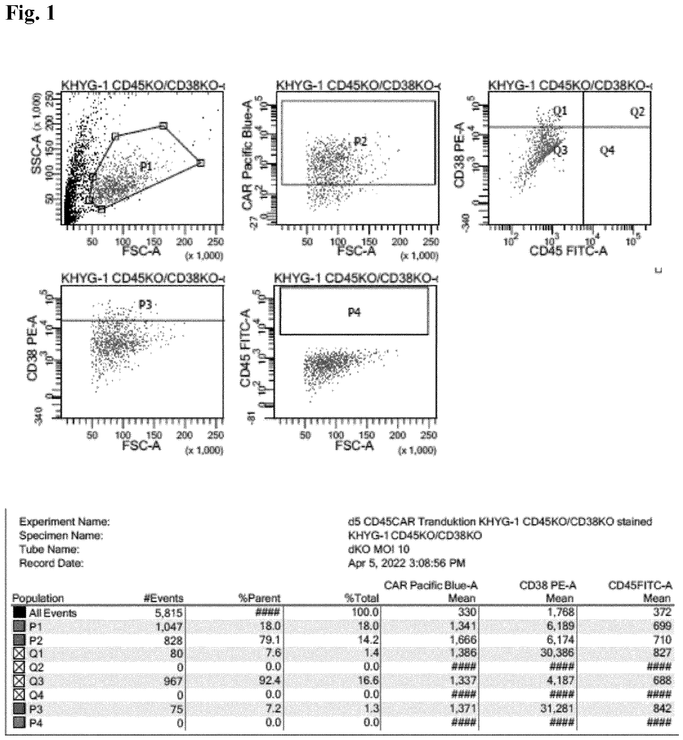

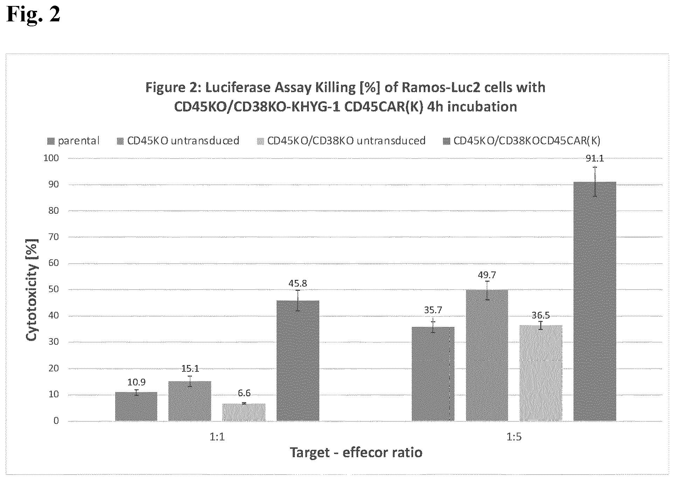

**Figure 1** shows results of flow cytometry after CD45KO, CD38KO, Fluorescence Activated Cell Sorting (FACS) for CD45⁻/CD38⁻ cells and CD45CAR transduction of KHYG-1 cells. Flow cytometry was performed on day 5 after CD45CAR transduction as described in example 3. As depicted, more than 90% of the resulting CD45CAR⁺ cells were found to be CD45KO/CD38KO. These cells were used for the cytotoxicity assay with results shown in figure 2
**Figure 2** shows the results from measuring the cytotoxic activity of CD45CAR-KHYG-1 cells with CD45/CD38 double KO against CD45-expressing Ramos cells in the 4h killing assay. It can be seen that the cytotoxicity is significantly stronger than in parental, unmodified as well as CAR-negative KHYG-1 cells with CD45 ± CD38 knockout. The cytotoxic effect is clearly dose-dependent.

### EXAMPLES

The present invention is further described in more detail by the following examples which are only provided for illustrating the invention and which are not to be construed as limiting the scope of the invention. The following material and methods were used in the Examples.

### Example 1:

For production of CD45KO/CD45CAR-KHYG-1 cells the CD45 gene in KHYG-1 cells was inactivated with CRISPR/CAS using electroporation as a transfection method as described in the co-pending European Patent application EP 22171675.6. Briefly, a CRISPRCas9-sgRNA RNP-complex was built by mixing the Cas9 protein TrueCut^{™} v2 (Thermofisher Scientific, Schwerte, Germany) with a sgRNA targeting the CD45 gene. The sgRNA was directed to nucleotides 261-279 (protospacer/crRNA) with the Protospacer adjacent motif (PAM) at nucleotides 280-282 of the CD45 gene. The CD45 gene is set forth in SEQ ID NO: 11 in EP 22171675.6. The sequence of nucleotides 261-282 of the CD45 gene are separately provided as SEQ ID NO: 12 in EP 22171675.6. The molar ratio was 1:2.5 of Cas9 to sgRNA in a total volume of approximately 2 µl. The mixture was incubated for 10 min at RT.

The KHYG-1 cells were prepared in a concentration of 400,000 cells/15 µl OPTI-MEM^{®} (Thermo Fisher Scientific) after a washing step (centrifugation 310 x g, 5 min, RT). 15 µl cells were added to the pre-built RNP-complexes and directly transferred to a 1-mm cuvette without causing any air bubbles. The cell-RNP mixture was electroporated with the GenePulser Xcell^{™} (BioRad, Munich, Germany) under the following conditions: 75 V, square-wave pulses, 10 ms pulse length, 3 pulses with 0,1 ms pause in between. The cells were washed out of the cuvette using pre-warmed KHYG-1 medium and cultured in 1 ml in a 24-well suspension plate (37 °C). To gain a pure CD45-negative KHYG-1 culture, the cells were further sorted after CD45-negative KHYG-1 with a FACS Cell Sorter after anti-CD45 antibody staining.

To generate CD45KO/CD38KO-CD45CAR-KHYG-1 cells, the CD38 gene was additionally inactivated in the former produced CD45KO-KHYG-1 cells using the method as described for the CD45KO but targeting CD38. The sequence of the target sequence in CD38 was 5'-TATGGCCAACTGCGAGTTC-3' (SEQ ID NO:11). The sequence of the spacer region (i.e., the gRNA target) used was as follows: 5'-GAACTCGCAGTTGGCCATA-3' (SEQ ID NO:12). Again, the cells were further sorted, now after CD38-negative KHYG-1 with a FACS Cell Sorter and anti-CD38 antibody staining.

Finally, the double-KO KHYG-1 cells were transduced inserting the CD45CAR with the transduction method as described in Example 2.

### Example 2:

The CD45KO/CD38KO-KHYG-1 cells were transduced with GALV-pseudotyped, γ-retroviral particles [24] to insert the gene encoding for the chimeric antigen receptor (CAR) (scFv anti-CD45) in conjunction with the BFP marker gene (for easy detection of transduced cells). One day before the transduction 6-well suspension plates were prepared by adding 2 ml Retronectin^{™} per well and incubation at 4°C overnight, or incubation for 2h at RT on the same day. On the day of transduction, the Retronectin^{™} was removed, 2 ml blocking buffer added per well (PBS + 2% BSA) and the plate incubated at RT for 30 minutes. The wells were washed twice with HBSS (1x) + 2.5% HEPES. Next, the supernatant containing the viral-vector particles harboring the CD45CAR vector (pRSF91_CD45-CD28c-T-CAR_iB_prePuroR) was added in TexMACS^{™} in a total volume of 1 ml per well to provide a multiplicity of infection (MOI) of 10. The plate was centrifuged for 1 h (1000 x g, 4°C) to pre-load vector particles to the Retronectin^{™}. Afterward, the medium containing non-bound vector particles was removed and 300,000 CD45KO/CD38KO-KHYG-1 cells were added in 2 ml KHYG-1 medium.

### Example 3:

The CD45KO/CD38KO-CD45CAR-KHYG-1 cells were analyzed using flow cytometry. The CD45CAR was detectable in the Pacific Blue channel via co-expressed blue-fluorescence protein (BFP). CD45 protein was stained with a monoclonal FITC-labelled antibody (CD45 anti-human, FITC, REAfinity^{™}, Miltenyi Biotec) and CD38 protein was stained with PE-labelled antibody (CD38 anti-human, PE, REAfinity^{™}, Miltenyi Biotec) according to the manufacturer protocol.

### Example 4:

The cells obtained from Examples 1 and 2 were used in a luciferase-based killing assay to test the killing ability of the CD45KO/CD38KO-CD45CAR-KHYG-1 cells. Ramos cells with luciferase activity were exploited as target cells after confirming their CD45 expression via antibody staining and flow cytometry.

The Ramos cell line was cultured in RPMI Medium 1640 (Gibco/Thermo Fisher Scientific, Schwerte Germany) supplemented with penicillin-streptomycin (100 U/ml and 100 µg/ml), L-glutamine (2 mM), 25 mM HEPES (1M), 13% heat-inactivated FCS, and 1 µg/ml puromycin. Cell cultures were provided fresh medium according to their needs and medium consumption. They were regularly split to ensure optimal density. Cell incubation was under standard conditions: 37°C, 100% relative humidity, 5% CO2.

On the day of the killing assay the CD45CAR-KHYG-1 cells were analyzed by flow cytometry to check for the rate of CAR+ cells based on BFP-expression. This rate was later used for calculation of the target effector ratios to assess Ramos:CD45CAR-KHYG-1 ratios.

The viability of the Ramos cells could be detected after addition of luciferin by measuring the bioluminescence with a plate reader. Target and effector cells were counted and seeded together in a white 96-well-OptiPlateTM (Perkin Elmer, Waltham, USA). 10,000 target cells were seeded in 100 µl/ well in effector cell medium, and effector cells were added at different effector:target (E-T) ratios. Effector cell medium was added to provide a total volume of 200 µL in each well. As zero control, "target cells only" were seeded in 200 µl effector cell medium to obtain the baseline luminescence value. As non-specific activity control, Ramos cells were cocultured with untransduced KHYG-1 cells in the same E-T ratios as for the CAR-transduced effector cells.

The plate was incubated at 37°C in the cell incubator for 4 h. After this, the plate was centrifuged at 1000 x g for 5 min at RT to pellet the cells. 100 µl supernatant were carefully re- moved with a multi-channel pipette. D-Luciferin Firefly (Biosynth, Staad, Switzerland) was diluted in PBS (10 ng in 100 µl PBS). Then, the cell pellets were resuspended in 100 µl of the D-Luciferin dilution per well and incubated for exactly 20 min in the dark. Luminescence was analyzed with the plate reader Infinite^{®}200 pro (Tecan, Männedorf, Switzerland) using 2000 ms integration time.

### LITERATURE

1. Schuster SJ, Bishop MR, Tam CS et al., Tisagenlecleucel in Adult Relapsed or Refractory Diffuse Large B-Cell Lymphoma. N Engl J Med. 2019; 380:45-56.
2. Neelapu SS, Locke FL, Bartlett NL, et al., Axicabtagene Ciloleucel CAR T-Cell Therapy in Refractory Large B-Cell Lymphoma. N Engl J Med. 2017;377:2531-2544.
3. Liu E, Marin D, Banerjee P, et al., Use of CAR-Transduced Natural Killer Cells in CD 19-Positive Lymphoid Tumors. N Engl J Med. 2020;382:545-553.
4. Raikar SS. Fleischer LC, Moot R, et al., Development of chimeric antigen receptors targeting T-cell malignancies using two structurally different anti-CD5 antigen binding domains in NK and CRISPR-edited T cell lines. Oncoimmunology. 2017;7:e1407898.
5. Gomes-Silva D, Srinivasan M. Sharma S et al., CD7-edited T cells expressing a CD7-specific CAR for the therapy of T-cell malignancies. Blood. 2017;130:285-296.
6. Rasaiyaah J, Georgiadis C. Preece R, Mock U. Qasim W. TCRaβ/CD3 disruption enables CD3-specific antileukemic T cell immunotherapy. JCI Insight. 2018 Jul 12;3(13):e99442.
7. Holmes N. CD45: all is not yet crystal clear. Immunology. 2006;117:145-55.
8. Bell GM. Dethloff GM, lmboden JB. CD45-negative mutants of a rat natural killer cell line fail to lyse tumor target cells. J Immunol. 1993;151:3646-53.
9. Hesslein DG, Takaki R, Hermiston ML, Weiss A, Lanier LL Dysregulation of signaling pathways in CD45-deficient NK cells leads to differentially regulated cytotoxicity and cytokine production. Proc Natl Acad Sci USA. 2006;103:7012-7.
10. Huntington ND, Xu Y, Nutt SL, Tarlinton DM. A requirement for CD45 distinguishes Ly49D-mediated cytokine and chemokine production from killing in primary natural killer cells J Exp Med. 2005;201:1421-33.
11. Rosen DB, Araki M, Hamerman JA, Chen T. Yamamura T, Lanier LL A Structural basis for the association of DAP12 with mouse, but not human. NKG2D. J Immunol. 2004; 173:2470-8.
12. Sparrow RL, McKenzie IF. A function for human T200 in natural killer cytolysis. Transplantation. 1983;36:166-71.
13. Starling GC, Davidson SE, McKenzie JL, Hart DN. Inhibition of natural killer-cell mediated cytolysis with monoclonal antibodies to restricted and non-restricted epitopes of the leucocyte common antigen. Immunology. 1987;61:351-6.
14. Starling GC. Hart DN. CD45 molecule cross-linking inhibits natural killer cell-mediated lysis independently of lytic triggering. Immunology. 1990;71:190-5.
15. Poggi A, Pardi R. Pella N et al., CD45-mediated regulation of LFA1 function in human natural killer cells. Anti-CD45 monoclonal antibodies inhibit the calcium mobilization induced via LFA1 molecules. Eur J Immunol. 1993;23:2454-63.
16. Ogilvy S, Louis-Dit-Sully C. Cooper J, Cassady RL, Alexander DR. Holmes N. Either of the CD45R and CD45RO isoforms are effective in restoring T cell. but not B cell. development and function in CD45-null mice. J Immunol. 2003;171:1792-800.
17. Martin SM, Mehta IK, Yokoyama WM, Thomas ML, Lorenz RG. Development of intestinal intraepithelial lymphocytes, NK cells, and NK 1.1+ T cells in CD45-deficient mice. J Immunol. 2001; 166:6066-73.
18. Godwin CD, McDonald GB, Walter RBSinusoidal obstruction syndrome following CD33-targeted therapy in acute myeloid leukemia. Blood. 2017; 129(16): 2330-2332.
19. Sun Y, Wang S, Zhao L, Zhang B, Chen H. FN-γ and TNF-α aggravate endothelial damage caused by CD123-targeted CART cell. Onco Targets Ther. 2019; 12:4907-4925.
20. Fernàndez JE, Deaglio S, Donati D, et al, Analysis of the distribution of human CD38 and of its ligand CD31 in normal tissues. J Biol Regul Homeost Agents. 1998; 12(3): 81-91.
21. Stikvoort A, van der Schans J, Sarkar S, Poels R, Ruiter R, Naik J, Yuan H, de Bruijn JD, van de Donk NWCJ, Zweegman S, Themeli M, Groen R, O'Dwyer M, Mutis T. CD38-specific Chimeric Antigen Receptor Expressing Natural Killer KHYG-1 Cells: A Proof of Concept for an "Off the Shelf" Therapy for Multiple Myeloma. Hemasphere. 2021 Jun 12;5(7):e596
22. Funaro, A.; De Monte, L.B.; Dianzani, U.; Forni, M.; Malavasi, F. Human CD38 is associated to distinct molecules which mediate transmembrane signaling in different lineages. Eur. J. Immunol. 1993, 23, 2407-2411
23. Sconocchia, G.; Titus, J.A.; Mazzoni, A.; Visintin, A.; Pericle, F.; Hicks, S.W.; Malavasi, F.; Segal, D.M. CD38 triggers cytotoxic responses in activated human natural killer cells. Blood 1999, 94, 3864-3871.
24. Mirow M, Schwarze LI, Fehse B, Riecken K. Efficient Pseudotyping of Different Retroviral Vectors Using a Novel, Codon-Optimized Gene for Chimeric GALV Envelope. Viruses. 2021;13:1471.
25. Hildinger M, Abel KL, OstertagW, Baum C. Design of 5' untranslated sequences in retroviral vectors developed for medical use. J Virol. 1999;73:4083-4089.
26. Hambach J, Riecken K, Cichutek S, Schütze K, Albrecht B, Petry K, Röckendorf JL Baum N, Kröger, N Hansen T, Schuch G, Haag F, Adam G, Fehse B, Bannas P, Koch-Nolte F. Targeting CD38-Expressing Multiple Myeloma and Burkitt Lymphoma Cells In Vitro with Nanobody-Based Chimeric Antigen Receptors (Nb-CARs). Cells 2020; 9: 321.
27. Lin Y, Pagel JM, Axworthy D, Pantelias A, Hedin N, Press OW. A genetically engineered anti-CD45 single-chain antibody-streptavidin fusion protein for pretargeted radioimmunotherapy of hematologic malignancies. Cancer Res. 2006; 66:3884-92.
28. Kundu S, Gurney M, O'Dwyer M. Generating natural killer cells for adoptive transfer: expanding horizons. Cytotherapy 2021, 23(7):559-566.
29. Meyer JE, Loff S, Dietrich J, Spehr J, Jurado Jiménez G, von Bonin M, Ehninger G, Cartellieri M, Ehninger A. Evaluation of switch-mediated costimulation in trans on universal CAR-T cells (UniCAR) targeting CD123-positive AML. Oncoimmunology 2021, 10(1):1945804.

## Claims

1. Modified natural killer (NK) cell, wherein said cell is **characterized by**
(a) the inactivation of the endogenous CD45 and CD38 genes,
(b) the expression of a chimeric antigen receptor (CAR) which is directed against CD45 and/or a CAR which is directed against CD38,
(c) the inability to proliferate,
wherein said cell exerts cytotoxicity against malignant cells upon its contacting with a malignant cell.

2. Modified NK cell of claim 1, wherein said NK cell is a human cell.

3. Modified NK cell of any of claims 1-2, wherein said NK cell comprises a point mutation in exon 7 of the p53 gene, wherein said point mutation preferably is a C to T at nucleotide 877 in codon 248 of the p53 gene.

4. Modified NK cell of any of claims 1-3, wherein said NK cell is a modified cell of the cell line KHYG-1, which has been deposited at the Leibniz-Institut DSMZ - Deutsche Sammlung von Mikroorganismen und Zellkulturen under accession number ACC 725, or a cell line derived from KHYG-1.

5. Modified NK cell of any of claims 1-4, wherein said NK cell expresses a CAR which is directed against CD45 and a CAR which is directed against CD38.

6. Modified NK cell of any of claims 1-5, wherein said NK cell expresses an additional CAR, wherein said additional CAR is directed to a protein selected from the group consisting of B Cell Maturation Antigen (BCMA), Kappa or lambda light chain, Lewis Y, CLL-1, G protein-coupled receptor, class C group 5 member D (GPRC5D), Fc receptor-homolog 5 (FcRH5), Human Semaphorin-4A (Sema4A)HLA-A1, HLA-A2, TCR alpha/beta, TCR gamma/delta, CD3, CD4, CD5, CD7, CD10, CD11a, CD13, CD16, CD19, CD20, CD22, CD23, CD26, CD30, CD33, CD34, CD37, CD38, CD43, CD44v6, CD45, CD56, CD59, CD79a, CD79b, CD90, CD117, CD123, CD133, CD138 and CD319, and wherein said additional CAR is most preferably directed to CD123 or Sema4A.

7. Modified NK cell of any of claims 1-6 for use in medicine.

8. Modified NK cell of any of claims 1-6 for use in a method of treating a cancer disease or an immunological disease in a patient, wherein said cancer disease is preferably selected from the group consisting of leukemia, lymphoma, myeloma, myelodysplastic syndromes, myeloproliferative neoplasms (MPN).

9. Modified NK cell for use in a method of claim 8, wherein said NK cell is autologous or allogeneic in respect of the patient to be treated.

10. Method of producing a NK cell which is unable to proliferate and exerts cytotoxicity against target cells, and preferably malignant cells, upon its contacting with the target cells, said method comprising
(a) providing an NK cell,
(b) inactivating the endogenous CD45 and CD38 genes of the NK cell,
(c) modifying the NK cell such that it expresses a CAR directed against CD45 and/or a CAR directed against CD38, and
(d) irradiating the NK cell such that it loses its ability to proliferate.

11. Method of claim 10, wherein irradiating the NK cell is performed at 5-50 Gray.

12. Method of any of claims 10-11, wherein inactivation step (b) can be performed either before, simultaneous with or after modification step (c), wherein inactivation step (b) is preferably performed before modification step (c), and wherein inactivation step (b) is most preferably performed 12-36 hours before modification step (c).

13. Method of any of claims 10-12, wherein
(i) inactivation step (b) is performed by targeted genome editing, and preferably by using CRISPR/CAS or TALENs; and/or
(ii) modification step (c) is performed with a viral or non-viral vector.

14. Method of any of claims 10-13, wherein the method further comprises step (e) in which the NK cell obtained after performing steps (a)-(c) or (a)-(d) is tested for CD45 and CD45-CAR expression and/or for CD38 and CD38-CAR expression.

15. Method of any of claims 10-14, wherein the method comprises modifying the NK cell such that it expresses both a CAR directed against CD45 and a CAR directed against CD38.
